# FASCICULE DE BREVET EUROPEEN

(11) **EP 3 603 415 B1**
(45) Date de publication et mention de la délivrance du brevet: **30.12.2020**
(21) Numéro de dépôt: 18306030.0
(22) Date de dépôt: 30.07.2018
(51) Int. Cl.: A23L 19/00, A61K 36/63, C07C 37/68

(54) **PROCEDE D'EXTRACTION DE COMPOSES PHENOLIQUES**
EXTRAKTIONSVERFAHREN VON PHENOLVERBINDUNGEN
METHOD FOR EXTRACTING PHENOLIC COMPOUNDS

(43) Date de publication de la demande: 05.02.2020
(73) Titulaire: Universite D'Avignon et Des Pays Du Vaucluse, 84029 Avignon (FR)
(72) Inventeur: TOMAO, Valérie, 84310 Morières les Avignon (FR); MALAPERT, Aurelia, 26110 Nyons (FR)
(74) Mandataire: Osha Liang

(56) Documents cités:
- CN-A- 103 948 933
- ES-A1- 2 293 824
- KR-A- 20030 019 760
- US-A1- 2010 204 179
- US-A1- 2011 086 815
- US-B2- 6 884 885

## Description

### DOMAINE TECHNIQUE

La présente description porte sur un procédé d'extraction d'au moins un composé phénolique contenu dans une phase aqueuse, notamment, mais pas exclusivement, d'origine végétale.

### ETAT DE L'ART

Les composés phénoliques sont valorisables notamment pour leurs propriétés antioxydantes et antibactériennes et peuvent montrer des activités biologiques ayant un impact bénéfique sur la santé humaine. Ces molécules interviennent de façon prépondérante dans la protection de l'organisme contre le développement des maladies cardiovasculaires et de certains cancers, notamment du fait de leur aptitude à piéger rapidement les espèces oxygénées réactives impliquées dans le développement de ces maladies.

En revanche, les composés phénoliques peuvent également être considérés comme des polluants lorsque ces composés sont présents dans des eaux usées industrielles ou encore l'eau du robinet. Dans les deux cas, qu'il s'agisse de valorisation ou d'éviter la pollution des eaux, l'exigence d'extraire ces composés contenus dans une phase aqueuse demeure.

Les procédés connus d'extraction de composés phénoliques contenus dans une phase aqueuse restent cependant insatisfaisants. Actuellement l'extraction de composés phénoliques d'une phase aqueuse peut être menée selon divers procédés dont les principaux intègrent des étapes de lyophilisation ou d'atomisation qui rendent les procédés complexes et longs et/ou l'instrumentation requise coûteuse. En outre, ces étapes entraînent de grandes variations de températures et en conséquence des coûts énergétiques élevés et peuvent générer de la dégradation ou de la perte de produit dans le cas de molécules thermosensibles.

Parmi les phases aqueuses d'origine végétale à partir desquelles l'on peut extraire des composés phénoliques, le broyât d'olive issu des moulins à huile ou encore les eaux de végétation de la production d'huile d'olive constituent des sources importantes de composés phénoliques d'intérêt, d'autant plus que la production d'oliviers et d'huile d'olive est très répandue autour de la Méditerranée et en constante augmentation.

Actuellement, la voie d'élimination la plus répandue pour le broyât d'olive issu des moulins à huile demeure l'épandage ou le compostage. Ces pratiques sont considérées aujourd'hui comme une source de pollution, due en particulier à la présence de composés phénoliques responsables de l'acidification des sols. L'application directe de ces molécules sur le sol peut engendrer des effets phytotoxiques, mais également une inhibition de la germination. D'autres techniques existent, comme la valorisation sous forme de biogaz, utilisable pour la production d'électricité et de chaleur, mais sont peu utilisées en raison du coût que ces techniques entraînent.

La disponibilité en produit de départ est pourtant garantie et la valorisation reste une problématique d'actualité.

Des complexes composé phénolique-cyclodextrine, plus spécifiquement des complexes d'inclusion dans lesquels un composé phénolique est encapsulé à l'intérieur de la cavité de la cyclodextrine, sont connus et ont été caractérisés en solution et à l'état solide. Par exemple, les documents García-Padial et al., Complexation of tyrosol with cyclodextrins, J. Incl. Phenom. Macrocylc. Chem., 75, pages 241-246, 2013, et López-García et al., Complexation of Hydroxytyrosol with β-cyclodextrins. An efficient photoprotection, Tetrahedron, 66, pages 8006-8011, 2010 décrivent ce type de complexes d'inclusion. La préparation de tels complexes est aussi décrite dans US 6 884 885 B2 et KR 2003 0019760 A, par exemple. Cependant, pour envisager un procédé avantageux sur le plan industriel, la possibilité de facilement séparer le produit formé est souvent une condition sine qua non, à laquelle aucun de ces documents ne satisfait. En effet, ces documents se limitent à la description de complexes d'inclusion de taille de l'ordre du nanomètre, présents à l'état liquide dans des solutions diluées.

### RESUME

Dans la présente description et dans les revendications, le terme « comprendre » est synonyme de (signifie la même chose que) « inclure », « contenir », et est inclusif ou ouvert et n'exclut pas d'autres éléments non décrits ou représentés. En outre, dans la présente description, le terme « environ » est synonyme de (signifie la même chose que) une marge inférieure et/ou supérieure de 10% de la valeur respective.

Un des objectifs de la présente description est de fournir un procédé d'extraction d'au moins un composé phénolique contenu dans une phase aqueuse.

Selon un premier aspect de la présente description, un tel objectif est atteint par un procédé comprenant les étapes suivantes :
- fournir une phase aqueuse ayant une concentration prédéterminée de composé(s) phénolique(s) ;
- ajouter au moins un saccharide en quantité prédéterminée à la phase aqueuse pour obtenir un mélange comprenant des agrégats solides de complexes composé phénolique-saccharide; et
- filtrer les agrégats solides de complexes composé phénolique-saccharide.
dans lequel :
- la concentration prédéterminée de composés phénoliques dans la phase aqueuse est au moins de 20 mM ;
- le pH de la phase aqueuse est acide ;
- la quantité prédéterminée de l'au moins un saccharide est d'au moins 4 équivalents molaires par équivalent molaire de l'au moins un composé phénolique ou de la somme de composés phénoliques ;
dans lequel le procédé est effectué à une température ne dépassant pas 40°C, et
dans lequel l'au moins un saccharide est une cyclodextrine.

Les complexes à l'état solide comprenant des saccharides et des composés phénoliques peuvent ainsi être récupérés. Dans le cadre de la présente invention, au moins un de ces saccharides est de la cyclodextrine.

Les inventeurs ont remarqué avec surprise qu'un tel procédé est particulièrement adapté à la séparation rapide et efficace de composés phénoliques provenant de n'importe quel type de phase aqueuse, ce qui en fait un procédé valorisable en vue de l'utilisation ultérieure de ces composés phénoliques, par exemple en agroalimentaire, en cosmétique ou encore dans le domaine médical, c'est-à-dire dans tous les domaines où les composés phénoliques peuvent être utilisés.

Dans les formes de réalisation de la présente description pour lesquelles les composés phénoliques extraits sont destinés à des applications où leurs propriétés bénéfiques pour la santé seront exploitées, comme par exemple en agroalimentaire, l'utilisation d'un saccharide pour piéger et extraire les composés phénoliques présente un avantage immédiat, dans la mesure où les saccharides présentent des propriétés organoleptiques acceptables, ce qui rend les complexes composé phénolique-saccharide formés directement valorisables.

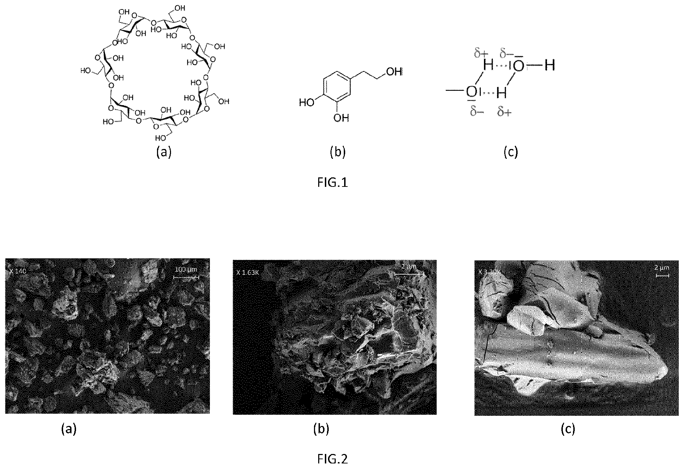

En outre, les complexes phénols-saccharide ont tendance à s'agréger et à former des agrégats solides, à savoir des particules comme on peut le voir, par exemple, sur les photos (a)-(c) contenues dans la FIG.2, pouvant être avantageusement filtrées par microfiltration classique.

Sans vouloir être liés par une quelconque théorie, les inventeurs estiment que la présence de groupements -OH provenant à la fois de l'au moins un saccharide et de l'au moins un composé phénolique, dans des conditions où la phase aqueuse a une concentration prédéterminée de composés phénoliques, entraîne la mise en jeu de forces d'attraction intermoléculaires entre l'au moins un saccharide, présent lui aussi en quantité déterminée, et l'au moins un composé phénolique. Ces forces d'attraction sont suffisantes pour favoriser des interactions dipolaires comme des liaisons hydrogènes (dont un exemple est représenté FIG.1(c)) qui sont relativement fortes (10 à 30 kJ.mol⁻¹). Ces interactions, nombreuses dans le mélange, pourraient être responsables de la cohésion des complexes composé phénolique-saccharide au sein des agrégats solides. Bien qu'il soit difficile d'établir un lien direct entre nombre de -OH portés par l'au moins un saccharide et le(s) composé(s) phénolique(s) et taille ou structure des agrégats solides résultants, les inventeurs ont constaté qu'une extraction rapide et efficace est possible en utilisant ces composés, i.e. au moins un composé phénolique et au moins un saccharide.

La concentration de l'au moins un composé phénolique dans la phase aqueuse avant l'addition d'au moins un saccharide est au moins de 20 mM. Selon une ou plusieurs formes de réalisation, elle peut être comprise entre 20 et 120 mM, par exemple entre 60 et 110 mM.

La quantité prédéterminée de l'au moins un saccharide est d'au moins 4 équivalents molaires, par exemple de 5 à 15 équivalents molaires de l'au moins un saccharide par équivalent molaire du composé phénolique ou de la somme de composés phénoliques contenus dans la phase aqueuse. La quantité de saccharide peut être comprise entre 8 et 12 équivalents molaires, par exemple entre 9 et 11 équivalents molaires.

Le pH de la phase aqueuse est acide, par exemple compris entre 1 et 6. De cette façon, les groupements hydroxyles provenant à la fois de l'au moins un saccharide et de l'au moins un composé phénolique sont sous leur forme protonée, propice à la formation d'interactions dipolaires décrites plus haut. Par exemple, lorsque la phase aqueuse est issue du traitement du broyat d'olive, le pH de la phase aqueuse peut être compris entre 2,2 et 6,0, ou en encore entre 4,7 et 5,7.

Le mélange entre saccharide(s), composé(s) phénolique(s) et phase aqueuse est effectué à température ambiante, *i.e.* à une température ne dépassant pas 40°C, par exemple de 15°C à 40°C, ou entre 18°C et 25 °C. Le mélange ne nécessite pas d'être chauffé, ce qui est très avantageux sur le plan industriel.

Selon une ou plusieurs formes de réalisation, le procédé est effectué à une température supérieure à 5°C, par exemple supérieure à 10°C.

Selon une ou plusieurs formes de réalisation, le procédé peut comprendre en outre l'agitation du mélange pour un temps prédéterminé jusqu'à l'obtention d'agrégats solides de complexes composé phénolique-saccharide. De cette façon, il est possible d'accélérer l'obtention d'agrégats solides de complexes composé phénolique-saccharide.

Selon une ou plusieurs formes de réalisation, le procédé comprend :
- la fourniture d'une phase aqueuse ayant une concentration prédéterminée de composé(s) phénolique(s) ;
- l'addition d'au moins un saccharide en quantité prédéterminée à la phase aqueuse pour obtenir un mélange comprenant la phase aqueuse, un ou plusieurs composés phénoliques et un ou plusieurs saccharides;
- l'agitation du mélange pour un temps prédéterminé jusqu'à l'obtention d'agrégats solides de complexes composé phénolique-saccharide, et
- la filtration des agrégats solides de complexes composé phénolique-saccharide.

Il est ici rappelé que, dans le cadre de la présente invention, ledit au moins un saccharide est une cyclodextrine.

Selon une ou plusieurs formes de réalisation, le ou les composés phénoliques contenus dans la phase aqueuse sont d'origine végétale. Les composés phénoliques peuvent, notamment mais pas exclusivement, être contenus dans une phase aqueuse issue du traitement du broyat d'olive, correspondant par exemple aux eaux de végétation du broyat d'olive, également appelées margines. Par exemple, une phase aqueuse riche en composés phénoliques contenus ou issus de la production d'huile d'olive, et adaptée à être soumise à un procédé selon une ou plusieurs formes de réalisation de la présente description peut comprendre de l'hydroxytyrosol (FIG.1(b)), des glucosides de l'hydroxytyrosol, du tyrosol, de l'acide caféique et/ou de l'acide *p*-coumarique.

Selon une ou plusieurs formes de réalisation, le procédé selon le premier aspect peut être adapté à des composés phénoliques de masse molaire plus importante, tels que, par exemple, les polyphénols.

Selon une ou plusieurs formes de réalisation, la phase aqueuse contenant l'au moins un composé phénolique n'est pas d'origine végétale. Une telle phase aqueuse peut provenir du traitement d'une eau usée et correspondre à l'eau du robinet ou être issue d'un procédé industriel (e.g. industrie papetière, production d'ammoniaque, raffinage du pétrole, etc.). Dans ces formes de réalisation, les composés phénoliques sont considérés comme des polluants, mais le procédé d'extraction selon la présente description est tout aussi applicable. Le procédé selon une ou plusieurs formes de réalisation de la présente description peut alors s'avérer très utile en vue de dépolluer une phase aqueuse.

Selon une ou plusieurs formes de réalisation, le mélange entre saccharide(s), composé(s) phénolique(s) et phase aqueuse est agité à température ambiante, *i.e.* à une température ne dépassant pas 40°C, par exemple de 15°C à 40°C, ou entre 18°C et 25 °C. Le mélange ne nécessite pas d'être chauffé, ce qui est très avantageux sur le plan industriel.

Selon une ou plusieurs formes de réalisation, le procédé est effectué à une température supérieure à 5°C, par exemple supérieure à 10°C.

Selon une ou plusieurs formes de réalisation, le mélange peut par exemple être agité dans un mélangeur comprenant un agitateur mécanique, par exemple tournant à une vitesse prédéterminée, par exemple comprise entre 10 et 1000 rpm, par exemple entre 150 et 300 rpm. Selon une ou plusieurs formes de réalisation, la durée d'agitation du mélange à température ambiante peut être prédéterminée, par exemple entre 0,5 h et 4 h, ou entre 0,5 h et 1,5 h. Une durée de contact inférieure à une heure peut généralement être suffisante pour cette étape du procédé.

Selon une ou plusieurs formes de réalisation, les agrégats solides de complexes composé phénolique-saccharide ont une taille égale ou supérieure à au moins 1 micron, par exemple d'au moins 2, 4 ou 10 microns. Par exemple, les agrégats solides de complexes composé phénolique-saccharide ont une taille d'au moins 4-6 microns.

Selon une ou plusieurs formes de réalisation, la filtration comprend une étape de microfiltration.

Selon une ou plusieurs formes de réalisation, la filtration comprend le passage du mélange à travers un filtre ayant une taille de pore comprise entre 1 micron et 10 microns, par exemple entre 2 microns ou 5 microns. Dans certaines formes de réalisation, la taille de pore du filtre est comprise entre 4 microns et 6 microns. Ainsi, contrairement à l'art antérieur qui déploie des moyens de séparation coûteux en temps et/ou en énergie car nécessitant des procédés complexes, les inventeurs ont constaté avec surprise qu'un procédé mettant enjeu une simple étape de microfiltration permet de séparer efficacement les complexes composé phénolique-saccharide de la phase aqueuse.

Selon la présente description, l'au moins un saccharide est une cyclodextrine, par exemple la béta-cyclodextrine (FIG.1, gauche). Les cyclodextrines permettent notamment de protéger les composés phénoliques de l'oxydation ou encore de masquer leur saveur grâce à leurs qualités organoleptiques. Selon une ou plusieurs formes de réalisation, l'au moins un saccharide peut comprendre ou consister en une molécule contenant un fragment de type saccharide de masse molaire comprise entre 5 et 100% de la masse molaire totale de la molécule, par exemple comprise entre 60 et 90 % de la masse molaire totale de la molécule.

Selon une ou plusieurs formes de réalisation le procédé peut comprendre une étape ultérieure optionnelle comprenant l'addition d'un alcool R-OH à l'agrégat solide de complexe composé phénolique-saccharide.

L'addition d'un alcool peut avoir pour effet de dissocier les agrégats et également les complexes composé phénolique-saccharide et donc de donner la possibilité de recycler avantageusement l'au moins un saccharide utilisé. Cette addition entraîne également l'obtention d'une solution organique riche en composés phénoliques, pouvant être utilisée en l'état, concentrée ou séchée jusqu'à l'obtention d'une poudre. L'alcool ajouté afin de décomposer les agrégats et dissocier les complexes composé phénolique-saccharide peut par exemple être un alcool aliphatique en C₁-C₈, linéaire ou branché, tel que l'éthanol ou le méthanol.

Selon une ou plusieurs formes de réalisation, la phase aqueuse contenant au moins un composé phénolique est obtenue par un procédé comprenant les étapes suivantes :
- la filtration, avec ou sans ajout de solvant, d'une source végétale contenant au moins un composé phénolique pour obtenir un filtrat de source végétale ;
- la concentration, par exemple sous pression réduite, par exemple comprise entre 150 mbar et 350 mbar à 40 °C, du filtrat de source végétale pour obtenir une solution de filtrat de source végétale;
- le dosage de complexation à l'aide du réactif de Folin Ciocalteu de la solution de filtrat de source végétale ; et
- la répétition des étapes de concentration et de dosage jusqu'à l'obtention d'une phase aqueuse ayant une concentration prédéterminée de composé(s) phénolique(s).

La filtration d'une source végétale peut être avantageusement effectuée sans ajout de solvant, et réalisée par exemple à l'aide d'un système de presse. La concentration du filtrat de source végétale peut être effectuée à l'aide d'un évaporateur, par exemple d'un évaporateur rotatif. Le dosage de complexation à l'aide du réactif de Folin Ciocalteu (ou dosage des composés phénoliques totaux) permet de mesurer la concentration en composés phénoliques de la solution de filtrat de source végétale.

Les formes de réalisation décrites ci-dessus ne sont pas exhaustives. Notamment, il est entendu que des formes de réalisation supplémentaires peuvent être envisagées sur la base de différentes combinaisons des formes de réalisation explicitement décrites. Sauf spécification contraire dans la présente description, il sera apparent pour l'homme du métier que toutes les formes de réalisation décrites ci-dessus peuvent être combinées entre elles. Par exemple, sauf spécification contraire, toutes les caractéristiques des formes de réalisation décrites ci-dessus, quelles que soient les formes de réalisation du procédé auxquelles elles se réfèrent, peuvent être combinées avec ou remplacées par d'autres caractéristiques d'autres formes de réalisation.

Des formes de réalisation selon les aspects référencés ci-dessus ainsi que des avantages supplémentaires apparaîtront à la lecture de la description détaillée suivante et des revendications annexées.

### BREVE DESCRIPTION DES DESSINS

La FIG. 1(a) est une représentation d'une structure moléculaire d'un exemple de saccharide, notamment la béta-cyclodextrine (a), qui peut être utilisée dans des exemples de réalisation du procédé de la présente description ;
LaFIG. 1(b) est une représentation d'une structure moléculaire d'un exemple de composé phénolique, notamment l'hydroxytyrosol (b), qui peut être utilisé dans des exemples de réalisation du procédé de la présente description ;
La FIG. 1(c) est une représentation d'un exemple d'interaction impliquée dans la formation d'agrégats solides de complexes composé phénolique-saccharide et mettant en jeu des liaisons hydrogène (c), qui peuvent être obtenus dans des exemples de réalisation du procédé de la présente description ; et
La FIG. 2 montre des photos (a)-(c) d'agrégats de complexes composé phénolique-saccharide qui peuvent être obtenus dans des exemples de réalisation du procédé de la présente description.

### DESCRIPTION DETAILLEE

Dans la description détaillée suivante des formes de réalisation de la présente invention, de nombreux détails spécifiques sont exposés afin de fournir une compréhension plus approfondie de la présente description. Cependant, il apparaîtra à l'homme du métier que la présente description peut être mise en œuvre sans ces détails spécifiques. Dans d'autres cas, des caractéristiques bien connues n'ont pas été décrites en détail pour éviter de compliquer inutilement la description.

### Exemple de procédé d'extraction d'au moins un composé phénolique contenu dans une phase aqueuse :

Selon un des modes de réalisation du procédé selon l'invention, le procédé s'applique à une phase aqueuse d'origine végétale, plus exactement une phase aqueuse correspondant aux eaux de végétation de la production d'huile d'olive, ou filtrat de broyat d'olive.

La préparation de la phase aqueuse est obtenue de la façon suivante : la matière première, à savoir le broyat d'olive, est tout d'abord filtrée par un système de presse sans ajout de solvant, puis concentrée sous vide à l'aide d'un évaporateur rotatif jusqu'à une concentration de 100 mM (i.e., 100 mM équivalent acide gallique, mesurée par dosage des composés phénoliques totaux à l'aide du réactif de Folin Ciocalteu), afin de constituer une fraction aqueuse riche en composés phénoliques de l'olive dont les majoritaires sont l'hydroxytyrosol et deux glucosides de l'hydroxytyrosol, le tyrosol, l'acide caféique et l'acide p-coumarique. La teneur de la phase aqueuse en ces composés est mesurée par chromatographie liquide haute performance avec détection à barrettes de photodiodes et spectrométrie de masse (UHPLC-DAD-MS) (Tableau I).

Ensuite, de la β-cyclodextrine est ajoutée à la phase aqueuse en quantité de 10 équivalents molaire par rapport à la concentration en composés phénoliques, pour obtenir un mélange comprenant des agrégats solides de complexes composé phénolique-β-cyclodextrine. Le mélange est alors agité pendant 1 h à 20°C, à la vitesse de 150 rpm.

Le mélange est transféré sur un filtre de porosité 5 (4 à 6µm). Les agrégats solides de complexes composé phénolique-β-cyclodextrine sont ainsi récupérés.

Les photos prise des agrégats formés par les complexes composé phénolique-cyclodextrine montrent une forte agrégation (FIG.2), et des tailles d'agrégats, de l'ordre de 100 microns de diamètre en moyenne, permettant leur microfiltration.

L'eau résiduelle contenue dans les agrégats récupérés par filtration est éliminée par séchage modéré dans une étuve ventilée. Comme le montre le Tableau I, le procédé permet d'extraire 20% des composés phénoliques d'intérêt majoritaires présents dans une phase aqueuse issue du traitement de broyat d'olive, parmi lesquels 96 % sont constitués de l'hydroxytyrosol, dérivés glucosides de l'hydroxytyrosol et tyrosol.

**Tableau I : Teneurs en composés phénoliques dans le filtrat de broyât d'olive et dans les poudres finales - Efficacités d'extraction.**

| | Filtrat de broyat d'olive (mg.L⁻¹) | Poudres finales (mg.L⁻¹) | Efficacité d'extraction (%) |
|---|---|---|---|
| Hydroxytyrosol | 1197,2 ± 17,5 | 189,3 ± 13,9 | 15,8 ± 1,2 |
| Hydroxytyroso-glucoside | 1170,2 ± 44,7 | 232,9 ± 15,4 | 19,9 ± 1,3 |
| Hydroxytyroso-glucoside isomère | 705,4 ± 43,1 | 133,7 ± 10,8 | 19,0 ± 1,5 |
| Tyrosol | 2534,1 ± 120,1 | 552,9 ± 46,9 | 21,8 ± 1,9 |
| Acide caféique | 107,3 ± 7,2 | 28,8 ± 3,6 | 26,8 ± 3,4 |
| Acide *p*-coumparique | 77,1 ± 3,9 | 24,8 ± 1,6 | 32,1 ± 2,1 |
| Total | 5792,0 ± 236,4 | 1162,3 ± 92,2 | 20,0 ± 0,8 |

## Revendications

1. Procédé d'extraction d'au moins un composé phénolique contenu dans une phase aqueuse, le procédé comprenant les étapes suivantes :
- fournir une phase aqueuse ayant une concentration prédéterminée de composé(s) phénolique(s) ;
- ajouter au moins un saccharide en quantité prédéterminée à la phase aqueuse pour obtenir un mélange comprenant des agrégats solides de complexes composé phénolique-saccharide; et
- filtrer les agrégats solides de complexes composé phénolique-saccharide ;
dans lequel :
- la concentration prédéterminée de composés phénoliques dans la phase aqueuse est au moins de 20 mM ;
- le pH de la phase aqueuse est acide ;
- la quantité prédéterminée de l'au moins un saccharide est d'au moins 4 équivalents molaires par équivalent molaire de l'au moins un composé phénolique ou de la somme de composés phénoliques ;
dans lequel le procédé est effectué à une température ne dépassant pas 40°C ; et
dans lequel l'au moins un saccharide est une cyclodextrine.

2. Procédé d'extraction selon la revendication 1, dans lequel l'au moins un composé phénolique est d'origine végétale.

3. Procédé d'extraction selon l'une quelconque des revendications précédentes, dans lequel le mélange est agité à une température ne dépassant pas 40°C.

4. Procédé d'extraction selon l'une quelconque des revendications précédentes, dans lequel la filtration comprend le passage du mélange à travers un filtre aux pores de taille comprise entre 1 à 10 µm.

5. Procédé d'extraction selon l'une quelconque des revendications précédentes, comprenant en outre :
- l'addition d'un alcool R-OH permettant la dissociation des agrégats et des complexes composé phénolique-saccharide en au moins un saccharide solide et une solution organique riche en composés phénoliques.

6. Procédé d'extraction selon la revendication 5, comprenant en outre :
- le recyclage de l'au moins un saccharide solide dans la phase aqueuse.

7. Procédé d'extraction selon la revendication 6, comprenant en outre :
- le séchage de la solution organique riche en composés phénoliques jusqu'à l'obtention d'une poudre.

8. Procédé d'extraction selon l'une quelconque des revendications précédentes, dans lequel la phase aqueuse contenant au moins un composé phénolique est obtenue par un procédé comprenant les étapes suivantes :
- la filtration d'une source végétale contenant au moins un composé phénolique pour obtenir un filtrat de source végétale ;
- la concentration du filtrat de source végétale pour obtenir une solution de filtrat de source végétale;
- le dosage des phénols complexés par le réactif de Folin Ciocalteu de la solution de filtrat de source végétale ; et
- la répétition des étapes de concentration et de dosage jusqu'à l'obtention d'une phase aqueuse ayant la concentration prédéterminée de composé(s) phénolique(s).

## Patentansprüche

1. Verfahren zur Extraktion wenigstens einer phenolischen Verbindung, die in einer wässrigen Phase enthalten ist, wobei das Verfahren die folgenden Schritte umfasst:
- Bereitstellen einer wässrigen Phase, die eine vorbestimmte Konzentration einer oder mehrerer phenolischer Verbindungen aufweist;
- Hinzufügen wenigstens eines Saccharids in vorbestimmter Menge zu der wässrigen Phase, um ein Gemisch zu erhalten, das feste Aggregate von Komplexen aus phenolischer Verbindung/Saccharid umfasst; und
- Abfiltrieren der festen Aggregate von Komplexen aus phenolischer Verbindung/Saccharid;
wobei
- die vorbestimmte Konzentration von phenolischen Verbindungen in der wässrigen Phase wenigstens 20 mM beträgt;
- der pH-Wert der wässrigen Phase im sauren Bereich liegt;
- die vorbestimmte Menge des wenigstens einen Saccharids wenigstens 4 Äquivalente pro Äquivalent der wenigstens einen phenolischen Verbindung oder der Summe der phenolischen Verbindungen beträgt;
wobei das Verfahren bei einer Temperatur von höchstens 40 °C durchgeführt wird; und
wobei das wenigstens eine Saccharid ein Cyclodextrin ist.

2. Extraktionsverfahren gemäß Anspruch 1, wobei die wenigstens eine phenolische Verbindung pflanzlicher Herkunft ist.

3. Extraktionsverfahren gemäß einem der vorstehenden Ansprüche, wobei das Gemisch bei einer Temperatur von höchstens 40 °C gerührt wird.

4. Extraktionsverfahren gemäß einem der vorstehenden Ansprüche, wobei die Filtration das Passieren des Gemischs durch einen Filter mit einer Porengröße im Bereich von 1 bis 10 µm umfasst.

5. Extraktionsverfahren gemäß einem der vorstehenden Ansprüche, weiterhin umfassend:
- die Zugabe eines Alkohols R-OH, der die Dissoziation der Aggregate und der Komplexe aus phenolischer Verbindung/Saccharid in wenigstens ein festes Saccharid und eine organische Lösung, die an phenolischen Verbindungen angereichert ist, erlaubt.

6. Extraktionsverfahren gemäß Anspruch 5, weiterhin umfassend:
- die cyclische Rückführung des wenigstens einen festen Saccharids in die wässrige Phase.

7. Extraktionsverfahren gemäß Anspruch 6, weiterhin umfassend:
- das Trocknen der an phenolischen Verbindungen angereicherten organischen Lösung bis zum Erhalt eines Pulvers.

8. Extraktionsverfahren gemäß einem der vorstehenden Ansprüche, wobei die wässrige Phase, die wenigstens eine phenolische Verbindung enthält, durch ein Verfahren erhalten wird, das die folgenden Schritte umfasst:
- Filtration einer pflanzlichen Quelle, die wenigstens eine phenolische Verbindung enthält, unter Erhalt eines Filtrats der pflanzlichen Quelle;
- Einengen des Filtrats der pflanzlichen Quelle unter Erhalt einer Filtratlösung der pflanzlichen Quelle;
- Bestimmen der komplexierten Phenole in der Filtratlösung der pflanzlichen Quelle durch das Folin-Ciocalteu-Reagens; und
- Wiederholung der Schritte des Einengens und Bestimmens bis zum Erhalt einer wässrigen Phase, die die vorbestimmte Konzentration der phenolischen Verbindung oder Verbindungen aufweist.

## Claims

1. Process for the extraction of at least one phenolic compound contained in an aqueous phase, the process comprising the following steps:
- providing an aqueous phase having a predetermined concentration of phenolic compound(s);
- adding at least one saccharide in a predetermined amount to the aqueous phase to obtain a mixture comprising solid aggregates of phenolic compound - saccharide complexes; and
- filtering the solid aggregates of phenolic compound - saccharide complexes;
wherein:
- the predetermined concentration of phenolic compounds in the aqueous phase is at least 20 mM;
- the pH of the aqueous phase is acidic;
- the predetermined amount of the at least one saccharide is at least 4 molar equivalents per molar equivalent of the at least one phenolic compound or the sum of phenolic compounds;
wherein the process is carried out at a temperature not exceeding 40°C; and
wherein the at least one saccharide is a cyclodextrin.

2. An extraction process according to claim 1, wherein the at least one phenolic compound is of plant origin.

3. An extraction process according to any one of the preceding claims, wherein the mixture is stirred at a temperature not exceeding 40°C.

4. An extraction process according to any one of the preceding claims, wherein the filtering includes passing the mixture through a filter with a pore size comprised between 1 and 10 µm.

5. An extraction process according to any one of the preceding claims, further comprising:
- adding an R-OH alcohol allowing the dissociation of aggregates and phenolic compound - saccharide complexes into at least one solid saccharide and an organic solution rich in phenolic compounds.

6. An extraction process according to claim 5, further comprising:
- recycling the at least one solid saccharide into the aqueous phase.

7. An extraction process according to claim 6, but also comprising the following step:
- drying the organic solution rich in phenolic compounds until a powder is obtained.

8. An extraction process according to any one of the preceding claims, wherein the aqueous phase containing at least one phenolic compound is obtained by a process comprising the following steps:
- filtering a plant source containing at least one phenolic compound to obtain a plant source filtrate;
- concentrating the plant source filtrate to obtain a plant source filtrate solution;
- dosing complexed phenols by the Folin Ciocalteu Reagent in the plant source filtrate solution; and
- repeating the concentrating and dosing steps until an aqueous phase having the predetermined concentration of phenolic compound(s) is obtained.
